# EUROPEAN PATENT APPLICATION

(11) **EP 3 563 896 A1**
(43) Date of publication of application: **06.11.2019**
(21) Application number: 18275174.3
(22) Date of filing: 07.11.2018
(51) Int. Cl.: A61M 21/00, A61M 16/00

(54) **DEVICES AND METHODS FOR ATTRACTING ENHANCED ATTENTION**

(71) Applicant: Thaler, Stephen L., St. Charles MO 63303 (US)
(72) Inventor: The designation of the inventor has not yet been filed
(74) Representative: Williams Powell

(57) **Abstract**

The present invention discloses devices and methods for attracting enhanced attention. Devices include: an input signal of a lacunar pulse train having characteristics of a pulse frequency of approximately four Hertz and a pulse-train fractal dimension of approximately one-half; and at least one controllable light source configured to be pulsatingly operated by the input signal; wherein a neural flame emitted from at least one controllable light source as a result of the lacunar pulse train is adapted to serve as a uniquely-identifiable signal beacon over potentially-competing attention sources by selectively triggering human or artificial anomaly-detection filters, thereby attracting enhanced attention.

## Description

### FIELD AND BACKGROUND OF THE INVENTION

The present invention relates to devices and methods for attracting enhanced attention. More specifically, the present invention relates to beacons for sustaining enhanced interest/attention, as well as to beacons with symbolic importance.

In the prior art, signal indicators and beacons are typically based upon color, brightness, periodic flashing frequency, rotational pattern, and motion, but not fractal dimension.

Both cognitive studies and simulations of the brain's limbo-thalamocortical system via artificial neural nets have shown that original ideas produced within the brain's stream of consciousness occur at a specific rhythm, typically near 4 hertz and a fractal dimension of approximately ½ (see Literature References below: Thaler, 1997b, 2013, 2014, 2016a, b, 2017b). An interval of 300 ms (∼ 4 Hz) has been referred to as the "speed of thought" (Tovée 1994).

In the referenced body of theoretical work of Thaler, the brain's thalamic reticular nucleus (TRN) is modeled as a constantly adapting auto-associative neural net (i.e., an anomaly or novelty detector), for which such ideational rhythms are the most noticeable due to their sporadic and unpredictable nature. Essentially, neural activation patterns within the cortex are thought to emit a telltale 'beacon' to the thalamus when they are generated within a stream having the above said frequency and fractal signature. Furthermore, these sporadic cognitive streams generally correspond to novel pattern formation and are considered the signature of inventive ideation.

It was also shown (Thaler 2016a) that the TRN's behavior as an anomaly detector was linked to creative thinking and enhanced attention in forming useful ideational patterns as stated in the following passage: "In the former case, creative achievements are the result of convergent thinking processes, requiring the attention of critic nets on the lookout for sporadic activations within the cortex that signal the formation of novel and potentially useful ideational patterns [3].With non-linear stimulus streams present in the external environment (i.e., sporadic events such as the two audible clicks used in EEG studies to measure so-called P50 response), the attention of critic nets selectively shifts to these sporadic external event streams [3,14] dominating within cortex, rather than mining the weaker, internally seeded stream of consciousness for seminal thought."

In another publication (Thaler 2016b), frequency and fractal dimension were shown to be indicative of the relation between attention, ideation novelty, and such thought-process characteristics: "The search for a suitable affordance to guide such attention has revealed that the rhythm of pattern generation by synaptically perturbed neural nets is a quantitative indicator of the novelty of their conceptual output, that cadence in turn characterized by a frequency and a corresponding temporal clustering that is discernible through fractal dimension."

Regarding human response to light modulation, the Color Usage Lab of the NASA Ames Research Center published related information dealing with "Blinking, Flashing, and Temporal Response" (https://colorusage.arc.nasa.gov/flashing_2.php), stating the following: "The rate of flashing has a powerful influence on the salience of flashing elements. The human eye is most sensitive to frequencies of 4-8 Hz (cycles/second). Very slow and very fast blinking are less attention-demanding than rates near that peak."

A proposed approach based on the effects of fractal flickering of light stimuli was previously published (Zueva 2013). Fractal flickering exhibits scale invariance with time on the evoked responses of the retina and visual cortex in normal and neurodegenerative disorders. In the proposed approach, standard stimuli are presented to patients who adapt to a flickering background with "specific chaotic interval variabilities between flashes (dynamic light fractal)." It was hypothesized that such an approach could be applied to facilitate adaptation to non-linear flickering with fractal dimensions in electrophysiological diagnostics.

Finally, in an article (Williams 2017) entitled, "Why Fractals Are So Soothing," related to fractal patterns in the paintings of Jackson Pollock, the physiological response to viewing images with fractal geometries having a fractal dimension of between 1.3 and 1.5 was suggested to be an "economical" means for the eye-tracking mechanism of the human visual system to simplify processing image content.

The ability to exploit fractal flickering for visual evoked responses (as in the approach described in Zueva 2013), or to detect a visually fractal image (as in the studies in Williams 2017) relate to visual and image processing.

It would be desirable to have devices and methods for attracting enhanced attention. Such devices and methods would, *inter alia,* provide unique advantages over the prior art mentioned above.

### SUMMARY

The present invention seeks to provide devices and methods for attracting enhanced attention.

It is noted that the term "exemplary" is used herein to refer to examples of embodiments and/or implementations, and is not meant to necessarily convey a more-desirable use-case. Similarly, the terms "alternative" and "alternatively" are used herein to refer to an example out of an assortment of contemplated embodiments and/or implementations, and is not meant to necessarily convey a more-desirable use-case. Therefore, it is understood from the above that "exemplary" and "alternative" may be applied herein to multiple embodiments and/or implementations. Various combinations of such alternative and/or exemplary embodiments are also contemplated herein.

Embodiments of the present invention provide a method for producing and providing a pulse train to an LED or lamp at a frequency and fractal dimension that is highly noticeable to humans, being the same rhythm with which original ideas are formed and recognized in both the brain and advanced Creativity Machines. A light source driven in such a manner may serve as an emergency beacon within environments filled with distracting light sources that are flickering randomly or periodically. Ease of detection may be improved using auto-associative neural nets as anomaly detectors within a machine-vision algorithm.

Thus, using TRN behavior as an anomaly filter in sustained creative activity and mental focus as detailed above in the context of the works of Thaler, the present invention exploits such a concept by embodying the same requisite characteristics (i.e., frequency and fractal dimension) in a signaling device in order to trigger the brain's innate ability to filter sensory information by "highlighting" certain portions in order to make those portions more noticeable to the brain.

That is, a single light-emitting element flashing at such a prescribed frequency is highly noticeable when viewed through anomaly detectors built from artificial neural networks. The sporadic nature of such pulse streams defeats the anomaly filter's ability to both learn and anticipate their rhythm, making said light pulses visible as anomalies. Additionally, in contrast to pulse trains, having fractal dimensions less than ½, the prescribed rhythms have sufficient frequency to catch the attention of a roving attention window, as when humans are shifting their attention across widely separated portions of a scene. If the detection system can calculate the fractal dimension of the anomalous light sources within the filtered scene, the "neural flame" may be used as an emergency beacon that discriminates itself from other alternating light sources within the environment.

Even to the naked eye, and without the use of an anomaly detector, fractal dimension ½ pulse streams preferentially attract the attention of human test subjects. The most attention-grabbing aspect of such streams is that the 'holes' or lacunarity between pulses occur as anomalies in what would otherwise be a linear stream of events. In other words, the pattern is frequently broken, such anomalous behavior possibly being detected by the TRN within the human brain as inconsistencies in the established arrival trend of visual stimuli. In contrast, should fractal dimension drop significantly below ½, the frequency of anomalous pulses drops, making them less noticeable to humans should either attention or gaze be wandering.

The incorporation of a "fractal rhythm" into a signal beacon, having a spatial fractal dimension near zero and a temporal delivery of a fractal dimension near ½, relates to exploiting the understanding of TRN behavior, thereby avoiding aspects of visual and image processing as contributing elements.

Embodiments of the present invention further provide a symbol celebrating the unique tempo by which creative cognition occurs. The algorithmically-driven neural flame may be incorporated within one or more structures that resemble candles or altar fixtures, for instance, to accentuate the light's spiritual significance. It is noted that that the light source or beacon can incorporate any type of light-emitting device.

Such embodiments stem from the notion of one perceiving neural net monitoring another imagining net, the so-called "Creativity Machine Paradigm" (Thaler 2013), which has been proposed as the basis of an "adjunct" religion wherein cosmic consciousness, tantamount to a deity, spontaneously forms as regions of space topologically pinch off from one another to form similar ideating and perceiving pairs, each consisting of mere inorganic matter and energy. Ironically, this very neural paradigm has itself proposed an alternative use for such a flicker rate, namely a religious object that integrates features of more traditional spiritual symbols such as candles and torches.

Moreover, in a theory of how cosmic consciousness may form from inorganic matter and energy (Thaler, 1997a, 2010, 2017), the same attentional beacons may be at work between different regions of spacetime. Thus, neuron-like, flashing elements may be used as philosophical, spiritual, or religious symbols, especially when mounted atop candle- or torch-like fixtures, celebrating what may be considered deified cosmic consciousness. Such a light source may also serve as a beacon to that very cosmic consciousness most likely operating via the same neuronal signaling mechanism.

Therefore, according to aspects of the present invention, there is provided for the first time a device for attracting enhanced attention, the device including: (a) an input signal of a lacunar pulse train having characteristics of a pulse frequency of approximately four Hertz and a pulse-train fractal dimension of approximately one-half; and (b) at least one controllable light source configured to be pulsatingly operated by the input signal; wherein a neural flame emitted from at least one controllable light source as a result of the lacunar pulse train is adapted to serve as a uniquely-identifiable signal beacon over potentially-competing attention sources by selectively triggering human or artificial anomaly-detection filters, thereby attracting enhanced attention.

Alternatively or additionally, the device further includes: (c) a processor for supplying the input signal of the lacunar pulse train having the characteristics; and (d) a digital-to-analog (D/A) converter for transmitting the input signal to at least one controllable light source.

More alternatively or additionally, the D/A converter is an onboard module of the processor, and wherein the module is embodied in at least one form selected from the group consisting of: hardware, software, and firmware.

More alternatively or additionally, the processor includes a thresholding unit for monitoring a random-walk trace for trace-axis crossings of a firing threshold of the thresholding unit, and wherein the trace-axis crossings result in activation transitions to generate pulse-activation sequences of the lacunar pulse train.

More alternatively or additionally, candidates of the pulse-activation sequences are filtered based on a zeroset dimension, and wherein the candidates are filled into a buffer of selected sequences having a fractal dimension of approximately one-half.

More alternatively or additionally, filtered patterns are randomly withdrawn from the selected sequences in the buffer, and wherein the filtered patterns are configured to serve as the input signal to the D/A converter for transmitting to at least one controllable light source.

Most alternatively or additionally, the filtered patterns are generated onboard the processor.

Alternatively or additionally, the uniquely-identifiable signal beacon reduces distraction by providing a preferential alert over the potentially-competing attention sources.

Alternatively or additionally, the neural flame serves as an object of contemplative focus embodying symbolic meaning of varying significance.

According to aspects of the present invention, there is provided for the first time a method for attracting enhanced attention, the method including the steps of: (a) generating a lacunar pulse train having characteristics of a pulse frequency of approximately four Hertz and a pulse-train fractal dimension of approximately one-half; (b) transmitting the input signal to at least one controllable light source; and (c) pulsatingly operating at least one controllable light source to produce a neural flame emitted from at least one controllable light source as a result of the lacunar pulse train is adapted to serve as a uniquely-identifiable signal beacon over potentially-competing attention sources by selectively triggering human or artificial anomaly-detection filters, thereby attracting enhanced attention.

Alternatively or additionally, the method further includes the step of: (d) monitoring a random-walk trace for trace-axis crossings of a firing threshold, and wherein the trace-axis crossings result in activation transitions to generate pulse-activation sequences of the lacunar pulse train.

More alternatively or additionally, the method further includes the steps of: (e) filtering candidates of the pulse-activation sequences based on a zeroset dimension; and (f) filling the candidates into a buffer of selected sequences having a fractal dimension of approximately one-half.

Most alternatively or additionally, the method further includes the steps of: (g) randomly withdrawing filtered patterns from the selected sequences in the buffer; and (h) using the filtered patterns as the input signal.

Alternatively or additionally, uniquely-identifiable signal beacon reduces distraction by providing a preferential alert over the potentially-competing attention sources.

Alternatively or additionally, neural flame serves as an object of contemplative focus embodying symbolic meaning of varying significance.

These and further embodiments will be apparent from the detailed description and examples that follow.

### BRIEF DESCRIPTION OF THE DRAWINGS

The present invention is herein described, by way of example only, with reference to the accompanying drawings, wherein:
Figure 1 is a simplified high-level schematic diagram depicting a neural-flame device for attracting enhanced attention, according to embodiments of the present invention;
Figure 2 is a simplified flowchart of the major process steps for operating the neural-flame device of Figure 1, according to embodiments of the present invention;
Figure 3 depicts a trace of the time evolution of input to a neuron-like thresholding unit of the neural-flame device of Figure 1, according to embodiments of the present invention;
Figure 4 depicts a video stream for detecting fractal beacons within a generalized scene from the neural-flame device of Figure 1, according to embodiments of the present invention.

### DESCRIPTION OF THE ILLUSTRATIVE EMBODIMENTS

The present invention relates to devices and methods for attracting enhanced attention. The principles and operation for providing such devices and methods, according to aspects of the present invention, may be better understood with reference to the accompanying description and the drawings.

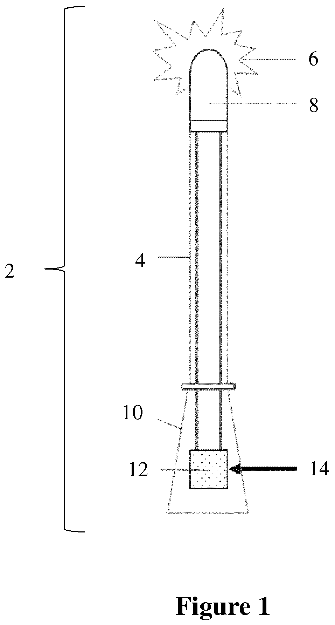

Referring to the drawings, Figure 1 is a simplified high-level schematic diagram depicting a neural-flame device for attracting enhanced attention, according to embodiments of the present invention. A neural-flame device **2** includes a support **4** serving as a beacon or an imitation candle, which may be configured to accommodate the needs of the application (regarding physical dimensions) such as an emergency alert or as an object of contemplative focus embodying varying significance.

Neural-flame device **2** has a controllable light source **6** (e.g., an LED component) with an optional translucent cover **8,** which can be shaped like a neuron's cell body or soma. Controllable light source **6** can incorporate any type of light-emitting device. Neural-flame device **2** includes a base **10** housing an optional digital-to-analog (D/A) converter (D/A module **12**) and an input connector **14** for supplying a digital input signal for driving controllable light source **6** with the required voltage sequence at a frequency corresponding to approximately 4 Hz and a fractal dimension near ½. It is noted that D/A module **12** can be implemented as hardware, software, and/or firmware as an integral component of a dedicated processor for neural-flame device **2.**

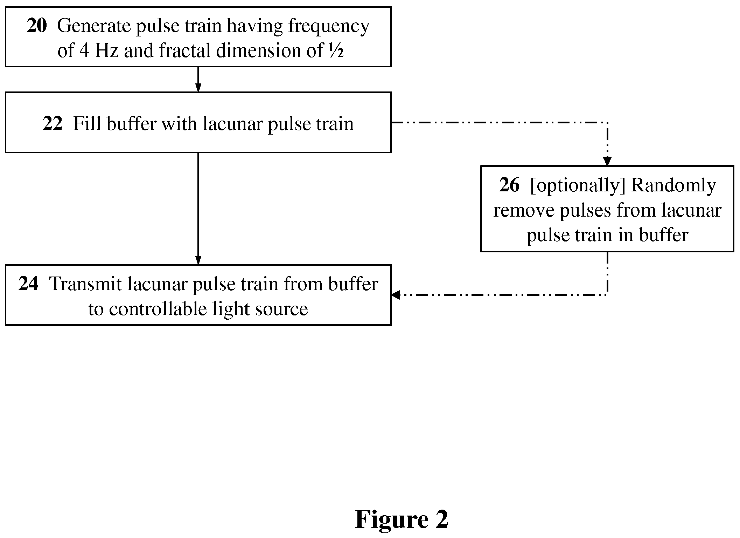

Figure 2 is a simplified flowchart of the major process steps for operating the neural-flame device of Figure 1, according to embodiments of the present invention. The process starts with the system generating pulse trains having a frequency of approximately 4 Hz and a fractal dimension of near ½ (Step **20**). A system buffer is then filled with these special lacunar pulse trains (Step **22**). These pulse trains are then sequentially withdrawn from the buffer, and then transmitted to controllable light source **6** via input connector **14** (Step **24**).

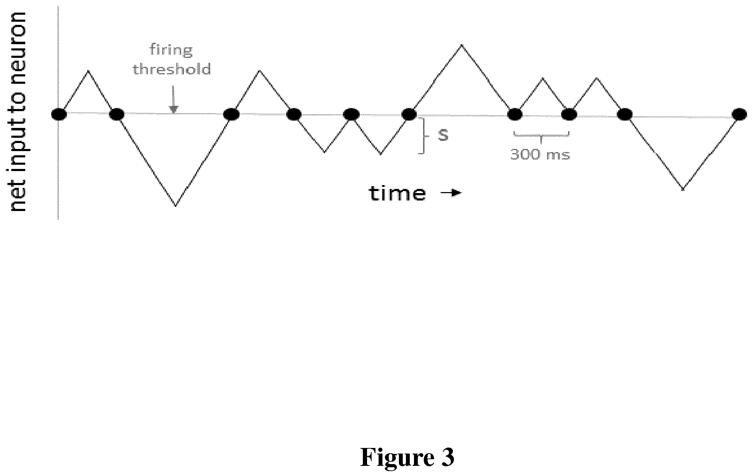

Optionally, pulse trains may be randomly removed from the buffer prior to transmitting the signal to controllable light source **6** (Step **26**). Such aspects are elaborated on in greater detail with regard to Figure 3.

Figure 3 depicts a trace of the time evolution of input to a neuron-like thresholding unit of the neural-flame device of Figure 1, according to embodiments of the present invention. The trace represents the output of a random-walk algorithm carried out on a computer or processor that is in turn applied to a neuron-like thresholding unit resulting in a series of activation transitions as the trace crosses (i.e., intersects) the "neuron's" firing threshold. The arrival patterns of these activation transitions are then filtered by an algorithm that calculates fractal dimension (i.e., zeroset dimension of the trace), and fills a buffer with those transition patterns having an approximate fractal dimension of ½. These filtered patterns are then withdrawn from the buffer, and transmitted to drive the controllable light source.

The algorithm may be generated in an onboard processor and power supply all within base **10** of neural-flame device **2.** It is noted that not only do such pulse patterns represent the desired 4 Hz, fractal dimension ½ pulse trains, but they largely differ from one another, thus preventing any anomaly detection filter, biological or not, from adapting to repeating activation streams.

The neuron-activation stream is generated by inputting a form of random walk of equal-sized steps to the neuron, with each such step being a notional 'coin flip' to determine whether the step is positive or negative in sign. As the random input crosses the neuron's firing threshold (as depicted in Figure 3), a pulse is triggered by the algorithm, the source of analog input to drive controllable light source **6** of neural-flame device **2.**

Returning to optional Step **26** of Figure 2, the resulting stream of the lacunar pulse train can be used as a set of candidate activation sequences that are then randomly withdrawn from the buffer, and transmitted to drive controllable light source **6.**

The random walk may be started repeatedly from zero in a series of trials, calculating fractal dimension for each, and then accumulating a library (i.e., a buffer) of just those short pulse sequences having the required fractal dimension near ½. Step **26** may be accomplished in nanoseconds, and the sequences computationally slowed to near 300-ms timescales prior to being transmitted to controllable light source **6.**

Other techniques may be employed as well to mitigate such effects, as known in the art. However, randomly withdrawing short pulse trains from the buffer has an advantage in that it adds another layer of randomness to the pulse train, allowing it to stand out when viewed through an anomaly detector, either in the brain or an artificial neural network-based novelty filter. With small pulse-train libraries, there is a chance of repetition as the short pulse trains are appended to each other, making it easier for the anomaly filter to adapt to them.

Such a "baseline reset" has been described (Thaler 2014). The fractal signature of the random walk is determined largely by its step size. In the case of the neural flame, the random walk is tuned to provide a trace (i.e., a wiggly line) that has a fractal dimension of 1.5. Sampling the crossings (i.e., intersections) of that trace with a baseline that is purposely introduced mid-channel yields a zeroset dimension of one less than that of the trace's fractal dimension, namely 0.5.

It is noted that the rigorous fractal dimension calculation (i.e., Mandelbrot Measures) is immune to the regions in which the trace departs from the baseline. Without directly viewing the trace, the zeroset dimension may be verified by waiting until the trace resumes its baseline crossings again, and then calculating how these intersections scale with time.

In Thaler 2014, the reset involves seeking the nearest memory to the network's current output pattern and using that as a new reference to measure how far that vector has walked. The equivalent of a single neuron's activation crisscrossing a baseline, the output pattern oscillates through a point in a multidimensional space.

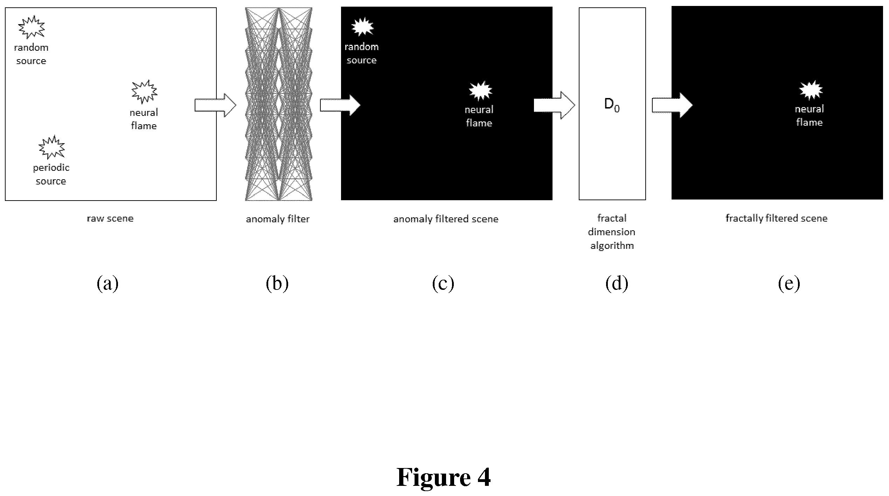

Figure 4 depicts a video stream for detecting fractal beacons within a generalized scene from the neural-flame device of Figure 1, according to embodiments of the present invention. Using a machine vision system, the video stream is propagated through an adaptive auto-associative neural net used as an anomaly filter. With periodic, random, and fractally-tuned beacons (as depicted in (a) "raw scene" of Figure 4), the anomaly filter (as in (b) of Figure 4) can block out the anomalies representing the periodic source (as in (c) of Figure 4). Subsequent algorithmic steps (as in (d) of Figure 4) calculate the fractal dimension of each anomaly's activation stream, enabling separation of any random source from that having a tuned fractal dimension (as in (e) of Figure 4). Thus, the use of fractal dimension at frequencies close to the clock cycle of the human brain, around 250-300 milliseconds, serves to enhance attention over other potentially-competing attention sources by selectively triggering the physiological anomaly-detection filtering of the brain.

To generate pulse trains to drive neural-flame device **2,** input to a computational neuron takes the form of a random walk over successive 300-millisecond intervals, each step being of equal magnitude (Figure 3). The aggregate intersections with the time axis represent the zeroset, with each of these points ultimately representing a pulse within the sequence driving neural-flame device **2.**

As these candidate pulse trains are generated, they are assessed for their zeroset (or fractal) dimension, D₀, which is approximated as: D₀ = ln(N₀)/ln(N), wherein N is the total number of 300 millisecond intervals sampled, and N₀ is the total number of intercepts of the neuron's net input with the firing threshold. As any new firing pattern is assessed with a fractal dimension near ½, the pattern is stored within a memory buffer or array. Subsequently, such pulse trains are randomly accessed and transmitted to D/A module **12** where they are converted to analog voltages to drive the neural flames of controllable light source **6.**

Alternatively, use of a storage buffer may be sidestepped by using an optimization algorithm that varies the step size of input variations to the neuron until the average fractal dimension of the pulse trains evaluate to the desired fractal dimension.

For use as a signal beacon, humans may search with or without the aid of a camera and machine-vision system. In the latter case, the camera's video stream may be viewed through an anomaly detector, the preferred embodiment being an adaptive auto-associative net that calculates the difference vector between the filter's input and output patterns, Δ**P** = **P**ᵢₙ - **P**_{**o**ut}, thus producing a map of anomalies within the camera's field of view. Subsequent filters then calculate the fractal dimension of anomalies appearing in this filtered view. Using such a methodology, not only can fractal dimension ½ sources be identified, but a range of prespecified fractal dimensions in the range (0, 1), opening a whole new approach to secure signaling and communication.

Furthermore, aspects of the present invention provide an object of contemplative focus embodying symbolic meaning of varying significance (e.g., philosophical/religious) due to the fact that the unique fractal rhythms used are those thought to: (1) be exploited by the brain to detect idea formation, and (2) have grandiose meaning as the temporal signature of creative cognition, whether in extraterrestrial intelligence or cosmic consciousness.

While the present invention has been described with respect to a limited number of embodiments, it will be appreciated that many variations, modifications, equivalent structural elements, combinations, sub-combinations, and other applications of the present invention may be made.

### LITERATURE REFERENCES

Tovée, MJ (1994). How fast is the speed of thought? Neuronal Processing, Current Biology, Vol. 4, No. 12, pp. 1125-1127.
Thaler, SL (1997a). The fragmentation of the universe and the devolution of consciousness, U.S. Library of Congress, Registration Number TXu000775586, January, 1997.
Thaler, SL (1997b). A quantitative model of seminal cognition: the creativity machine paradigm, Proceedings of the Mind II Conference, Dublin, Ireland, 1997.
Thaler, SL (2010). Thalamocortical Algorithms in Space! The Building of Conscious Machines and the Lessons Thereof, In the Proceedings of World Future 2010: Sustainable Futures, Strategies, and Technologies, July 8-10, 2010, Boston, MA.
Thaler, SL (2013). The Creativity Machine Paradigm, Encyclopedia of Creativity, Invention, Innovation, and Entrepreneurship, (ed.) E.G. Carayannis, Springer Science+Business Media, LLC.
Zueva, MV (2013). Dynamic Fractal Flickering as a Tool in Research of Non-Linear Dynamics of the Evoked Activity of a Visual System and the Possible Basis for New Diagnostics and Treatment of Neurodegenerative Diseases of the Retina and Brain, World Appl. Sci. J., 27 (4): 462-468, 2013.
Thaler, SL (2014). Synaptic Perturbation and Consciousness, International Journal of Machine Consciousness, Vol. 06, No. 02, pp. 75-107.
Thaler, SL (2016a). Cycles of Insanity and Creativity within Contemplative Neural Systems, Medical Hypotheses, 94:138-147, Elsevier, 2016.
Thaler, SL (2016b). Pattern Turnover within Synaptically Perturbed Neural Systems, Procedia Computer Science, 88, Elsevier, 2016.
Thaler, SL and Zbikowski, K. (2017b). Cognitive Engines Contemplating Themselves, APA Newsletter on Philosophy and Computers, 17(1), Fall 2017.
Williams, F (2017). Why Fractals Are So Soothing, The Atlantic, Jan. 26, 2017.

## Claims

1. A device for attracting enhanced attention, the device comprising:
(a) an input signal of a lacunar pulse train having characteristics of a pulse frequency of approximately four Hertz and a pulse-train fractal dimension of approximately one-half; and
(b) at least one controllable light source configured to be pulsatingly operated by said input signal;
wherein a neural flame emitted from said at least one controllable light source as a result of said lacunar pulse train is adapted to serve as a uniquely-identifiable signal beacon over potentially-competing attention sources by selectively triggering human or artificial anomaly-detection filters, thereby attracting enhanced attention.

2. The device of claim 1, the device further comprising:
(c) a processor for supplying said input signal of said lacunar pulse train having said characteristics; and
(d) a digital-to-analog (D/A) converter for transmitting said input signal to said at least one controllable light source.

3. The device of claim 2, wherein said D/A converter is an onboard module of said processor, and wherein said module is embodied in at least one form selected from the group consisting of: hardware, software, and firmware.

4. The device of claim 3, wherein said processor includes a thresholding unit for monitoring a random-walk trace for trace-axis crossings of a firing threshold of said thresholding unit, and wherein said trace-axis crossings result in activation transitions to generate pulse-activation sequences of said lacunar pulse train.

5. The device of claim 4, wherein candidates of said pulse-activation sequences are filtered based on a zeroset dimension, and wherein said candidates are filled into a buffer of selected sequences having a fractal dimension of approximately one-half.

6. The device of claim 5, wherein filtered patterns are randomly withdrawn from said selected sequences in said buffer, and wherein said filtered patterns are configured to serve as said input signal to said D/A converter for transmitting to said at least one controllable light source.

7. The device of claim 6, wherein said filtered patterns are generated onboard said processor.

8. The device of any one of claims 1 to 7, wherein said uniquely-identifiable signal beacon reduces distraction by providing a preferential alert over said potentially-competing attention sources.

9. The device of any one of claims 1 to 7, wherein said neural flame serves as an object of contemplative focus embodying symbolic meaning of varying significance.

10. A method for attracting enhanced attention, the method comprising the steps of:
(a) generating a lacunar pulse train having characteristics of a pulse frequency of approximately four Hertz and a pulse-train fractal dimension of approximately one-half;
(b) transmitting said input signal to at least one controllable light source; and
(c) pulsatingly operating said at least one controllable light source to produce a neural flame emitted from said at least one controllable light source as a result of said lacunar pulse train is adapted to serve as a uniquely-identifiable signal beacon over potentially-competing attention sources by selectively triggering human or artificial anomaly-detection filters, thereby attracting enhanced attention.

11. The method of claim 10, the method further comprising the step of:
(d) monitoring a random-walk trace for trace-axis crossings of a firing threshold, and wherein said trace-axis crossings result in activation transitions to generate pulse-activation sequences of said lacunar pulse train.

12. The method of claim 11, the method further comprising the steps of:
(e) filtering candidates of said pulse-activation sequences based on a zeroset dimension; and
(f) filling said candidates into a buffer of selected sequences having a fractal dimension of approximately one-half.

13. The method of claim 12, the method further comprising the steps of:
(g) randomly withdrawing filtered patterns from said selected sequences in said buffer; and
(h) using said filtered patterns as said input signal.

14. The method of any one of claims 10 to 13, wherein said uniquely-identifiable signal beacon reduces distraction by providing a preferential alert over said potentially-competing attention sources.

15. The method of any one of claims 10 to 13, wherein said neural flame serves as an object of contemplative focus embodying symbolic meaning of varying significance.

## Amended claims

### Amended claims in accordance with Rule 137(2) EPC.

1. A device (2) for attracting enhanced attention, the device comprising:
(a) an input signal of a lacunar pulse train having characteristics of a pulse frequency of approximately four Hertz and a pulse-train fractal dimension of approximately one-half generated from a random walk over successive 300 millisecond intervals, each step being of equal magnitude and representative of a pulse train satisfying a fractal dimension equation of ln(number of intercepts of a neuron's net input with a firing threshold)/ln(the total number of 300 ms intervals sampled); and
(b) at least one controllable light source (6) configured to be pulsatingly operated by said input signal;
wherein a neural flame is emitted from said at least one controllable light source as a result of said lacunar pulse train.

2. The device of claim 1, the device (2) further comprising:
(c) a processor for supplying said input signal of said lacunar pulse train having said characteristics; and
(d) a digital-to-analog (D/A) converter (12) for transmitting said input signal to said at least one controllable light source (6).

3. The device of claim 2, wherein said D/A converter (12) is an onboard module of said processor, and wherein said module is embodied in at least one form selected from the group consisting of: hardware, software, and firmware.

4. The device of claim 3, wherein said processor includes a thresholding unit for monitoring a random-walk trace for trace-axis crossings of a firing threshold of said thresholding unit, and wherein said trace-axis crossings result in activation transitions to generate pulse-activation sequences of said lacunar pulse train.

5. The device of claim 4, wherein candidates of said pulse-activation sequences are filtered based on a zeroset dimension, and wherein said candidates are filled into a buffer of selected sequences having a fractal dimension of approximately one-half.

6. The device of claim 5, wherein filtered patterns are randomly withdrawn from said selected sequences in said buffer, and wherein said filtered patterns are configured to serve as said input signal to said D/A converter for transmitting to said at least one controllable light source.

7. The device of claim 6, wherein said filtered patterns are generated onboard said processor.

8. A method for attracting enhanced attention, the method comprising the steps of:
(a) generating a lacunar pulse train having characteristics of a pulse frequency of approximately four Hertz and a pulse-train fractal dimension of approximately one-half generated from a random walk over successive 300 millisecond intervals, each step being of equal magnitude and representative of a pulse train satisfying a fractal dimension equation of ln(number of intercepts of a neuron's net input with a firing threshold)/ln(the total number of 300 ms intervals sampled);
(b) transmitting said input signal to at least one controllable light source (6); and
(c) pulsatingly operating said at least one controllable light source (6) to produce a neural flame emitted from said at least one controllable light source (6) as a result of said lacunar pulse train.

9. The method of claim 8, the method further comprising the step of:
(d) monitoring a random-walk trace for trace-axis crossings of a firing threshold, and wherein said trace-axis crossings result in activation transitions to generate pulse-activation sequences of said lacunar pulse train.

10. The method of claim 9, the method further comprising the steps of:
(e) filtering candidates of said pulse-activation sequences based on a zeroset dimension; and
(f) filling said candidates into a buffer of selected sequences having a fractal dimension of approximately one-half.

11. The method of claim 10, the method further comprising the steps of:
(g) randomly withdrawing filtered patterns from said selected sequences in said buffer; and
(h) using said filtered patterns as said input signal.
